(19) Europäisches Patentamt European Patent Office Office européen des brevets

(11) **EP 3 034 515 B1**

(12) **EUROPEAN PATENT SPECIFICATION**

(45) Date of publication and mention of the grant of the patent:
**29.08.2018  Bulletin 2018/35**

(51) Int Cl.:
*C07K 19/00* [(2006.01)]    *C07K 14/375* [(2006.01)]
*C07K 14/765* [(2006.01)]    *A61K 38/16* [(2006.01)]
*A61P 35/00* [(2006.01)]    *A61P 7/00* [(2006.01)]

(21) Application number: **14854197.2**

(22) Date of filing: **11.07.2014**

(86) International application number:
**PCT/CN2014/082031**

(87) International publication number:
**WO 2015/055026 (23.04.2015 Gazette 2015/16)**

(54) **FUSION PROTEIN OF GANODERMA LUCIDUM IMMUNOREGULATORY PROTEIN AND HUMAN SERUM ALBUMIN AND ITS USE FOR TREATING CANCER**

FUSIONSPROTEIN AUS DEM IMMUNREGULATORISCHEN PROTEIN GANODERMA LUCIDUM UND HUMANEM SERUMALBUMIN UND DESSEN VERWENDUNG IN DER BEHANDLUNG VON KREBS

PROTÉINE DE FUSION ASSOCIANT LA PROTÉINE IMMUNORÉGULATRICE DE GANODERMA LUCIDUM ET LA SÉRUMALBUMINE HUMAINE ET SON UTILISATION POUR LE TRAITMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2013  CN 201310479016**

(43) Date of publication of application:
**22.06.2016  Bulletin 2016/25**

(73) Proprietors:
• **Zhang, Xitian**
  **Huangpu District**
  **Shanghai 200032 (CN)**
• **Sun, Fei**
  **Huangpu District**
  **Shanghai 200032 (CN)**

(72) Inventors:
• **LIANG, Chongyang**
  **Shanghai 200032 (CN)**
• **ZHANG, Xitian**
  **Shanghai 200032 (CN)**
• **Sun, Fei**
  **Shanghai 200032 (CN)**

(74) Representative: **Sun, Yiming**
  **HUASUN Patent- und Rechtsanwälte**
  **Friedrichstraße 33**
  **80801 München (DE)**

(56) References cited:
WO-A1-2010/135854    WO-A1-2013/067355
CN-A- 101 463 089    CN-A- 102 274 487
KR-A- 20120 079 492    US-A1- 2004 063 635
US-A1- 2009 053 173

• YU , DAN.: 'Preliminary Study on Recombinant protein Ganoderma Lucidum Immunoregulatory (rLZ-8) anti-mouse ascites hepatoma cell line S1800 part of search.' CHINESE MASTER'S THESES FULL-TEXT DATABASE (ELECTRONIC JOURNALS), MEDICINE AND HEALTH SCIENCES. 15 September 2009, pages E072 - 131, XP008181821
• ZHOU, HUI. MEDICINE AND HEALTH SCIENCES 15 August 2013, XP008181822
• LEI, J.Y. ET AL.: 'Expression, purification and characterization of recombinant human interleukin-2-serum albumin (rhIL-2-HSA) fusion protein in Pichia pastoris.' PROTEIN EXPRESSION AND PURIFICATION vol. 84, no. 1, 17 May 2012, pages 154 - 160, XP028493249

SEQUENCE LISTING

<110> ZHANG, Xitian; SUN, Fei

<120> Fusion protein of recombinant ganoderma immunoregulatory protein and human serum albumin, preparation method thereof, and application thereof

<130> PCT14119

<150> 201310479016.0
<151> 2013-10-15

<160> 9

<170> PatentIn version 3.3

<210> 1
<211> 725
<212> PRT

<213> rLZ-8-HSA

<400> 1

```
Ser Asp Thr Ala Leu Ile Phe Arg Leu Ala Trp Asp Val Lys Lys Leu
 1               5                  10                  15

Ser Phe Asp Tyr Thr Pro Asn Trp Gly Arg Gly Asn Pro Asn Asn Phe
             20                  25                  30

Ile Asp Thr Val Thr Phe Pro Lys Val Leu Thr Asp Lys Ala Tyr Thr
         35                  40                  45

Tyr Arg Val Ala Val Ser Gly Arg Asn Leu Gly Val Lys Pro Ser Tyr
     50                  55                  60

Ala Val Glu Ser Asp Gly Ser Gly Lys Val Asn Phe Leu Glu Tyr Asn
 65                  70                  75                  80

Ser Gly Tyr Gly Ile Ala Asp Thr Asn Thr Ile Gly Val Phe Val Val
             85                  90                  95

Asp Pro Asp Thr Asn Asn Asp Phe Ile Ile Ala Gly Trp Asn Gly Gly
             100                 105                 110

Gly Gly Ser Ser Met Lys Trp Val Thr Phe Ile Ser Leu Leu Phe Leu
         115                 120                 125

Phe Ser Ser Ala Tyr Ser Arg Gly Val Phe Arg Arg Asp Ala His Lys
     130                 135                 140

Ser Glu Val Ala His Arg Phe Lys Asp Leu Gly Glu Glu Asn Phe Lys
145                 150                 155                 160

Ala Leu Val Leu Ile Ala Phe Ala Gly Tyr Leu Gly Gly Cys Pro Phe
             165                 170                 175

Glu Asp His Val Lys Leu Val Asn Glu Val Thr Glu Phe Ala Lys Thr
         180                 185                 190

Cys Val Ala Asp Glu Ser Ala Glu Asn Cys Asp Lys Ser Leu His Thr
         195                 200                 205
```

```
Leu Phe Gly Asp Lys Leu Cys Thr Val Ala Thr Leu Arg Glu Thr Tyr
210             215             220

Gly Glu Met Ala Asp Cys Cys Ala Lys Gly Glu Pro Glu Arg Asn Glu
225             230             235             240

Cys Phe Leu Gly His Lys Asp Asp Asn Pro Asn Leu Pro Arg Leu Val
                245             250             255

Arg Pro Glu Val Asp Val Met Cys Thr Ala Phe His Asp Asn Glu Glu
                260             265             270

Thr Phe Leu Lys Lys Tyr Leu Tyr Glu Ile Ala Arg Arg His Pro Tyr
                275             280             285

Phe Tyr Ala Pro Glu Leu Leu Phe Phe Ala Lys Arg Tyr Lys Ala Ala
                290             295             300

Phe Thr Glu Cys Cys Gly Ala Ala Asp Lys Ala Ala Cys Leu Leu Pro
305             310             315             320

Lys Leu Asp Glu Leu Arg Asp Glu Gly Lys Ala Ser Ser Ala Lys Gly
                325             330             335

Arg Leu Lys Cys Ala Ser Leu Gly Lys Phe Gly Glu Arg Ala Phe Lys
                340             345             350

Ala Trp Ala Val Ala Arg Leu Ser Gly Arg Phe Pro Lys Ala Glu Phe
                355             360             365

Ala Glu Val Ser Lys Leu Val Thr Asp Leu Thr Lys Val His Thr Glu
                370             375             380

Cys Cys His Gly Asp Leu Leu Glu Cys Ala Asp Asp Arg Ala Asp Leu
385             390             395             400

Ala Lys Tyr Ile Cys Glu Asn Gly Asp Ser Ile Ser Ser Lys Leu Lys
                405             410             415

Glu Cys Cys Glu Lys Pro Leu Leu Glu Lys Ser His Cys Ile Ala Glu
                420             425             430

Val Glu Asn Asp Glu Met Pro Ala Asp Leu Pro Ser Leu Ala Ala Asp
                435             440             445

Phe Val Glu Ser Lys Asp Val Cys Lys Asn Tyr Ala Glu Ala Lys Asp
450             455             460

Val Phe Leu Gly Met Phe Leu Tyr Glu Tyr Ala Arg Arg His Pro Asp
465             470             475             480

Tyr Ser Val Val Leu Leu Leu Arg Leu Ala Lys Thr Tyr Glu Thr Thr
                485             490             495

Leu Glu Lys Cys Cys Ala Ala Ala Asp Pro His Glu Cys Tyr Ala Lys
                500             505             510

Val Phe Asp Glu Phe Lys Pro Leu Val Glu Glu Pro Gly Asn Leu Ile
                515             520             525

Lys Gly Asn Cys Glu Leu Phe Glu Gly Leu Gly Glu Tyr Lys Phe Gly
                530             535             540
```

```
Asn Ala Leu Leu Val Arg Tyr Thr Lys Lys Val Pro Gly Val Ser Thr
545                 550             555             560

Pro Thr Leu Val Glu Val Ser Arg Asn Leu Gly Lys Val Gly Ser Lys
                565             570             575

Cys Cys Lys His Pro Glu Ala Lys Arg Met Pro Cys Ala Glu Asp Tyr
            580             585             590

Leu Ser Val Val Leu Asn Gly Leu Cys Val Leu His Glu Lys Thr Pro
            595             600             605

Val Ser Asp Arg Val Thr Lys Cys Cys Thr Glu Ser Leu Val Asn Arg
        610             615             620

Arg Pro Cys Phe Ser Ala Leu Glu Val Asp Glu Thr Tyr Val Pro Lys
625             630             635             640

Glu Phe Asn Ala Glu Thr Phe Thr Phe His Ala Asp Ile Cys Thr Leu
                645             650             655

Ser Glu Lys Glu Arg Gly Ile Lys Lys Gly Thr Ala Leu Val Glu Leu
            660             665             670

Val Lys His Lys Pro Lys Ala Thr Lys Glu Gly Leu Lys Ala Val Met
        675             680             685

Asp Asp Phe Ala Ala Phe Val Glu Lys Cys Cys Lys Ala Asp Asp Lys
        690             695             700

Glu Thr Cys Phe Ala Glu Glu Gly Lys Lys Leu Val Ala Ala Ser Gly
705             710             715             720

Ala Ala Leu Gly Leu
            725
```

<210> 2
<211> 350
<212> PRT
<213> rLZ-8-FcI

<400> 2

```
Arg Pro Ser Asp Thr Ala Leu Ile Phe Arg Leu Ala Trp Asp Val Lys
1               5               10              15

Lys Leu Ser Phe Asp Tyr Thr Pro Asn Trp Gly Arg Gly Asn Pro Asn
            20              25              30

Asn Phe Ile Asp Thr Val Thr Phe Pro Lys Val Leu Thr Asp Lys Ala
        35              40              45

Tyr Thr Tyr Arg Val Ala Val Ser Gly Arg Asn Leu Gly Val Lys Pro
        50              55              60

Ser Tyr Ala Val Glu Ser Asp Gly Ser Gly Lys Val Asn Phe Leu Glu
65              70              75              80

Tyr Asn Ser Gly Tyr Gly Ile Ala Asp Thr Asn Thr Ile Gly Val Phe
                85              90              95
```

```
        Val Val Asp Pro Asp Thr Asn Asn Asp Phe Ile Ile Ala Gly Trp Asn
                    100             105             110

        Gly Gly Gly Gly Ser Ser Glu Pro Lys Ser Cys Asp Lys Thr His Thr
                    115             120             125

        Cys Pro Pro Cys Pro Ala Pro Glu Leu Leu Gly Gly Pro Ser Val Phe
            130             135             140

        Leu Phe Pro Pro Lys Pro Lys Asp Thr Leu Met Ile Ser Arg Thr Pro
        145             150             155             160

        Glu Val Thr Cys Val Val Val Asp Val Ser His Glu Asp Pro Glu Val
                    165             170             175

        Lys Phe Asn Trp Tyr Val Asp Gly Val Glu Val His Asn Ala Lys Thr
                    180             185             190

        Lys Pro Arg Glu Glu Gly Tyr Asn Ser Thr Tyr Arg Val Val Ser Val
                    195             200             205

        Leu Thr Val Leu His Gly Asp Trp Leu Asn Gly Lys Glu Tyr Lys Cys
            210             215             220

        Lys Val Ser Asn Lys Ala Leu Pro Ala Pro Ile Glu Lys Thr Ile Ser
        225             230             235             240

        Lys Ala Lys Gly Gly Pro Arg Glu Pro Gly Val Tyr Thr Leu Pro Pro
                    245             250             255

        Ser Arg Glu Glu Met Thr Lys Asn Gly Val Ser Leu Thr Cys Leu Val
                    260             265             270

        Lys Gly Phe Tyr Pro Ser Asp Ile Ala Val Glu Trp Glu Ser Asn Gly
                    275             280             285

        Gly Pro Glu Asn Asn Tyr Lys Thr Thr Pro Pro Val Leu Asp Ser Asp
            290             295             300

        Gly Ser Phe Phe Leu Tyr Ser Lys Leu Thr Val Asp Lys Ser Arg Trp
        305             310             315             320

        Gly Gly Gly Asn Val Phe Ser Cys Ser Val Met His Glu Ala Leu His
                    325             330             335

        Asn His Tyr Thr Gly Lys Ser Leu Ser Leu Ser Pro Gly Lys
                    340             345             350
```

<210> 3
<211> 507
<212> PRT
<213> rLZ-8-Fc4

<400> 3

```
        Arg Pro Ser Asp Thr Ala Leu Ile Phe Arg Leu Ala Trp Asp Val Lys
        1               5               10              15

        Lys Leu Ser Phe Asp Tyr Thr Pro Asn Trp Gly Arg Gly Asn Pro Asn
                    20              25              30
```

```
Asn Phe Ile Asp Thr Val Thr Phe Pro Lys Val Leu Thr Asp Lys Ala
        35                  40                  45

Tyr Thr Tyr Arg Val Ala Val Ser Gly Arg Asn Leu Gly Val Lys Pro
        50                  55                  60

Ser Tyr Ala Val Glu Ser Asp Gly Ser Gly Lys Val Asn Phe Leu Glu
65                  70                  75                  80

Tyr Asn Ser Gly Tyr Gly Ile Ala Asp Thr Asn Thr Ile Gly Val Phe
                85                  90                  95

Val Val Asp Pro Asp Thr Asn Asn Asp Phe Ile Ile Ala Gly Trp Asn
            100                 105                 110

Gly Gly Gly Gly Ser Ser Tyr Thr Gly Arg Phe Lys Asp Lys Ala Lys
            115                 120                 125

Leu Thr Ala Val Thr Ser Ala Asn Thr Ala Tyr Met Glu Leu Ser Ser
    130                 135                 140

Leu Thr Asn Glu Asp Ser Ala Val Tyr Tyr Cys Ser Ile Ile Tyr Phe
145                 150                 155                 160

Asp Tyr Ala Asp Phe Ile Met Asp Tyr Trp Gly Gly Gly Thr Thr Val
                165                 170                 175

Thr Val Ser Thr Ala Ser Thr Lys Gly Pro Ser Val Phe Pro Leu Ala
            180                 185                 190

Pro Cys Ser Arg Ser Thr Ser Glu Ser Thr Ala Ala Leu Gly Cys Leu
            195                 200                 205

Val Lys Asp Tyr Phe Pro Glu Pro Val Thr Val Ser Trp Asn Ser Gly
    210                 215                 220

Ala Leu Thr Ser Gly Val His Thr Phe Pro Ala Val Leu Gly Xaa Ser
225                 230                 235                 240

Gly Leu Tyr Ser Leu Ser Ser Val Val Thr Val Pro Ser Ser Ser Leu
                245                 250                 255

Gly Thr Lys Thr Tyr Thr Cys Asn Val Asp His Lys Pro Ser Asn Thr
            260                 265                 270

Lys Val Asp Lys Arg Val Glu Ser Lys Tyr Gly Pro Pro Cys Pro Ser
            275                 280                 285

Cys Pro Ala Pro Glu Phe Leu Gly Gly Pro Ser Val Phe Leu Phe Pro
    290                 295                 300

Pro Lys Pro Lys Asp Thr Leu Met Ile Ser Arg Thr Pro Glu Val Thr
305                 310                 315                 320

Cys Val Val Val Asp Val Ser Gly Glu Asp Pro Glu Val Gly Phe Asn
            325                 330                 335

Trp Tyr Val Asp Gly Val Glu Val His Asn Ala Lys Thr Lys Pro Arg
            340                 345                 350

Glu Glu Gly Phe Asn Ser Thr Tyr Arg Val Val Ser Val Leu Thr Val
            355                 360                 365
```

```
Leu His Gly Asp Trp Leu Xaa Gly Lys Glu Tyr Lys Cys Lys Val Ser
    370             375             380

Xaa Lys Gly Leu Pro Ser Ser Ile Glu Lys Thr Ile Ser Xaa Ala Xaa
385             390             395             400

Gly Gly Pro Arg Glu Pro Gly Val Tyr Thr Leu Pro Pro Ser Gly Glu
            405             410             415

Glu Met Thr Lys Asn Gly Val Ser Leu Thr Cys Leu Val Lys Gly Phe
            420             425             430

Tyr Pro Ser Asp Ile Ala Val Glu Trp Glu Ser Asn Gly Gly Pro Glu
            435             440             445

Asn Asn Tyr Lys Thr Thr Pro Pro Val Leu Asp Ser Asp Gly Ser Phe
    450             455             460

Phe Leu Tyr Ser Arg Leu Thr Val Asp Lys Ser Xaa Trp Gly Glu Gly
465             470             475             480

Asn Val Phe Ser Cys Ser Val Met His Glu Ala Leu His Asn His Tyr
            485             490             495

Thr Gly Lys Ser Leu Ser Leu Ser Leu Gly Lys
            500             505
```

<210> 4
<211> 25
<212> DNA
<213> Artificial Sequence

<220>
<223> RT-PCR forward primer

<400> 4
cataggcctt ctgatactgc tttga      25

<210> 5
<211> 23
<212> DNA
<213> Artificial Sequence

<220>
<223> RT-PCR reverse primer

<400> 5
cggggtaccg aattcctatt aca      23

<210> 6
<211> 25
<212> DNA
<213> Artificial Sequence

<220>
<223> RT-PCR forward primer

<400> 6
gttaggcctt ctgatactgc tttga      25

<210> 7
<211> 23
<212> DNA
<213> Artificial Sequence

<220>
<223> RT-PCR reverse primer

<400> 7
tagggtacct catttaccag ggg          23

<210> 8
<211> 22
<212> DNA
<213> Artificial Sequence

<220>
<223> RT-PCR forward primer

<400> 8
ccgaggcctt ctgatactgc tt          22

<210> 9
<211> 23
<212> DNA
<213> Artificial Sequence

<220>
<223> RT-PCR reverse primer

<400> 9
gatggtacct cacggagcat gag          23

## Description

## BACKGROUND OF THE PRESENT INVENTION

### Field of Invention

The present invention relates to construction, expression, purification and application of a recombinant fusion protein, and more particularly to a preparation method and an application of a fusion protein of a recombinant *ganoderma* immunoregulatory protein and a human serum albumin, which is used for cure diseases such as leukopenia and tumor caused by chemotherapy.

### Description of Related Arts

*Ganoderma* immunoregulatory protein (LZ-8) comes from a mycelium of *ganoderma tsugae.* A structure of the recombinant *ganoderma* immunoregulatory protein comprises: an important N-terminal domain for forming a dimer and a C-terminal FNIII domain; wherein the N-terminal domain of the rLZ-8 comprises an α-helix and a β-strand, an α-helix and a β-strand of an N-terminal of an LZ-8 monomer form an important dumbbell-shaped dimer binding domain through space exchanging with the respective domains of another LZ-8. It has been reported that the LZ-8 has biological activity on immuloregulation (referring to Chinese patent CN201110222012.5) and killing tumor cells (referring to Chinese patent ZL200810050206.X).

Conventionally, main reasons for a short half-life of protein drugs are: 1) hydrolysis is one of the metabolism methods for most proteins, and existence of protein enzymes in body tissues reduces the activity of the protein; 2) kidney is the most important organ in the process of breakdown and metabolism of small-molecular protein; most proteins, with relative molecular weight of less than 69000Da, are able to be excreted by glomerulus filtration. 3) liver plays an important role in the process of protein drug metabolism, wherein drug is delivered to liver cells through diffusion and carrier transport, and then is degraded by microsomal enzyme P450, protease or lysosome in cytosol; peptides and proteins with larger molecules are absorbed by the liver cells through endocytosis mediated by a receptor before being degraded by liver cells. A molecular weight of the dimer is less than 26kDa; a clearance rate may be high; and pharmacokinetic parameters are difficult to meet requirements of pharmaceutical developments. Therefore, only by extending duration of the LZ-8 in vivo by gene-level fusion and other technical methods can solid foundations be laid for clinical application. In order to prolong the half-life of the protein drugs, in recent years, researchers mainly studies from aspects such as albumin fusion, chemical modification, microencapsulation, construction of mutants, and glycosylation. As the research progresses, new varieties of long-acting protein drugs are emerging.

Gene fusion technology connects different genes, for expressing a fusion protein with complex functions. Through gene fusion technology, molecular weights of the peptide and protein drugs are increased or affinity between the drug and the receptor is changed, so as to extend the half-life of drugs. Principle of constructing the fusion protein are as follows: removing a stop codon of a coding gene of a protein, and then connecting a coding gene of another protein with a stop codon, so as to realize fusion of the coding genes of the two proteins, for simultaneously expressing two proteins. The fusion protein gene has high stability and is able to be regulated and expressed, product thereof is homogeneous, affects on activity of protein and peptide drugs is small, etc., which conventionally provide a sufficient method for studying long-acting peptide and protein drugs.

Conventionally, widely studied fusion genes are human serum albumin gene, human immunoglobulin (IgG4, IgG1) gene, etc. Human serum albumin (HSA for short) is a protein in human plasma, non-glycosylated single-chain polypeptide thereof comprises 585 amino acids, and a molecular weight is 66kDa. A concentration of HSA in plasma is 42g/L, which is about 60% of total plasma proteins. Human serum albumin in the body fluids is able to transport fatty acids, bile pigments, amino acids, steroid hormones, metal ions, different therapeutic molecules, etc; while normal blood pressure is maintained. Clinically, human serum albumin is applicable to treatment of shock and burn, and is able to be used as a supplement to blood loss caused by surgery, accident or bleeding; human serum albumin is also able to be used as plasma compatibilizer.

Human immunoglobulin (IgG) is the most abundant protein in human blood, whose half-life is 21 days. It has been reported that a fusion of an Fc fragment of IgG and other proteins is able to significantly increase the biological activity and half life of other proteins in vivo, wherein most researchers choose IgG1 and IgG4 Fc fragments as a fusion object. The method has been widely used in some clinically important cell factors and soluble receptors, such as sTNF-αR, LFA3, CTLA-4, IL-2, and IFN-α, with significant success.

In view of the above background technology, the present invention uses the gene recombination technology to respectively fuse *ganoderma* immunoregulatory protein with human serum albumin and Fc fragment of human immunoglobulin IgG, so as to construct a eukaryotic expression system. After expression and purification, a target protein is obtained, and related biological activity and pharmaceutical researches are provided to the target protein. Result shows that the *ganoderma* immunoregulatory protein (LZ-8) is significantly different from the HSA fusion protein in pharmaceutical and biological activities as well as pathological application.

**SUMMARY OF THE PRESENT INVENTION**

The present invention is defined by the claims. An object of the present invention is to provide a fusion protein of a recombinant *ganoderma* immunoregulatory protein and a human serum albumin, and a preparation method thereof, in such a manner that applications thereof in curing diseases such as leukopenia caused by chemotherapy and anti-tumor drugs are studied.

A fusion protein sequence: according to the present invention, the fusion protein rLZ-8-HSA of a *ganoderma* immunoregulatory protein (LZ-8) and an HSA comprises the *ganoderma* immunoregulatory protein and the HSA; an amino acid sequence thereof is:
SDTALIFRLAWDVKKLSFDYTPNWGRGNPNNFIDTVTFPKVLTDKAYTYRVAVSGRNLGVKPSYAVESDGSQKVNFL
EYNSGYGIADTNTIQVFVVDPDTNNDFIIAQWNGGGGSSMKWVTFISLLFLFSSAYSRGVFRRDAHKSEVAHRFKDLG
EENFKALVLIAFAQYLQQCPFEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAPELLFFAKRYKAAFTECCQA
ADKAACLLPKLDELRDEGKASSAKQRLKCASLQKFGERAFKAWAVARLSQRFPKAEFAEVSKLVTDLTKVHTECCHG
DLLECADDRADLAKYICENQDSISSKLKECCEKPLLEKSHCIAEVENDEMPADLPSLAADFVESKDVCKNYAEAKDVFL
GMFLYEYARRHPDYSVVLLLRLAKTYETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCAEDYLSWLNQLCVLHEKTPVSDRVTKCCTESLVN
RRPCFSALEVDETYVPKEFNAETFTFHADICTLSEKERQIKKQTALVELVKHKPKATKEQLKAVMDDFAAFVEKCCKAD
DKETCFAEEGKKLVAASQAALGL (SEQ ID NO. 1), wherein an amino acid sequence of a connecting peptide connecting a C-terminal of the *ganoderma* immunoregulatory protein and the human serum albumin is GGGGSS. In one instance, a fusion protein rLZ-8-Fc1 of the *ganoderma* immunoregulatory protein (LZ-8) and a human IgG1Fc fragment comprises the *ganoderma* immunoregulatory protein and the IgG1Fc fragment; wherein an amino acid sequence thereof is:
RPSDTALIFRLAWDVKKLSFDYTPNWGRGNPNNFIDTVTFPKVLTDKAYTYRVAVSGRNLGVKPSYAVESDGSQKVN
FLEYNSGYGIADTNTIQVFWDPDTNNDFIIAQWNGGGGSSEPKSCDKTHTCPPCPAPELLGGPSVFLFPPKPKDTLMI
SRTPEVTCVWDVSHEDPEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPEN-
NYKTTPPVLDSDGSFFLYSKLTVDKSRWQQGNVFSCSVMHEALHNHYTQKSLSLSPGK (SEQ ID NO. 2), wherein the amino acid sequence of the connecting peptide connecting the C-terminal of the *ganoderma* immunoregulatory protein and the human serum albumin is GGGGSS. In yet another instance, a fusion protein rLZ-8-Fc4 of the *ganoderma* immunoregulatory protein (LZ-8) and a human IgG4Fc fragment comprises the *ganoderma* immunoregulatory protein and the IgG4Fc fragment; wherein an amino acid sequence thereof is:

RPSDTALIFRLAWDVKKLSFDYTPNWGRGNPNNFIDTVTFPKVLTDKAYTYRVAVSGR

NLGVKPSYAVESDGSQKVNFLEYNSGYGIADTNTIQVFVVDPDTNNDFIIAQWNGGGG

SSYTQRFKDKAKLTAVTSANTAYMELSSLTNEDSAVYYCSIIYFDYADFIMDYWGQGTT

VTVSTASTKGPSVFPLAPCSRSTSESTAALGCLVKDYFPEPVTVSWNSGALTSGVHTF

PAVLQXSGLYSLSSVVTVPSSSLGTKTYTCNVDHKPSNTKVDKRVESKYGPPCPSCPA

PEFLGGPSVFLFPPKPKDTLMISRTPEVTCVVVDVSQEDPEVQFNWYVDGVEVHNAKT

KPREEQFNSTYRVVSVLTVLHQDWLXGKEYKCKVSXKGLPSSIEKTISXAXGQPREPQ

VYTLPPSQEEMTKNQVSLTCLVKGFYPSDIAVEWESNGQPENNYKTTPPVLDSDGSFF

LYSRLTVDKSXWQEGNVFSCSVMHEALHNHYTQKSLSLSLGK      (SEQ   ID   NO.   3),

wherein the amino acid sequence of the connecting peptide connecting the C-terminal of the *ganoderma* immunoregulatory protein and the human serum albumin is GGGGSS.

Construction of engineering strains, as well as expression and purification of a target protein are as follows: respectively establishing carriers of nucleotide sequences of an LZ-8 fusion protein; using electricity transformation technology to obtain a host cell with Pichia pastoris as a fusion protein extracellular expression; wherein fermentation conditions of Pichia pastoris are optimized and expression value of the fusion protein is improved; purifying a target product by methods such as gravity column affinity chromatography, a molecular sieve, immobilized metal chelate affinity chromatography (IMAC) method, hydrophobic interaction chromatography (HIC), anion exchange chromatography, wherein purities of different fusion proteins obtained are above 99%, which provides crude drugs for subsequent pharmacological experiments.

Pharmacy experiments are as follows: using the different fusion proteins as contrasts of rLZ-8 for activity experiments, wherein through an ELISA method, a test result illustrates that a biological activity of the fusion protein rLZ-8-HSA is higher than a biological activity of the rLZ-8 which is not fused, and there is a significant difference, which is statistically significant; however, biological activities of the rLZ-8-Fc1 and the rLZ-8-Fc4 are lower than an activity index of the rLZ-8 which is not fused, and there is a significant difference; using the different fusion proteins as contrasts of the rLZ-8 for in vivo half-life experiments, wherein according to the ELISA method, in vivo half-lives of the different fusion proteins are all significantly improved to about 3 times of the one of the rLZ-8; meanwhile, using the different fusion proteins as contrasts of the rLZ-8 for leucopenia treating experiments, and counting white blood cells with an animal whole blood cell analyzer, wherein a test result illustrates that there is significant difference between the different fusion proteins and the rLZ-8 in treatment of the leukopenia; wherein with a same dosage, the fusion protein rLZ-8-HSA has a shortest cycle for promoting leukocyte growth; and with a same treatment cycle, the fusion protein rLZ-8-HSA promotes more white blood cells to grow. Therefore, the HSA is more suitable as a fusion partner of the rLZ-8, which is able to significantly enhance application of the LZ-8 in leukopenia treatment. According to melanoma cell growth inhibition experiments of the fusion protein rLZ-8-HSA, it is shown that with a same dosage (in LZ-8 meter), the fusion protein rLZ-8-HSA effectively inhibits melanoma cells growth, which is significantly different from a treatment effect of the rLZ-8 which is not fused. Meanwhile, according to a preferred embodiment of the fusion protein rLZ-8-HSA for inhibition of liver tumor cell growth, it can be seen that in a same treatment cycle, a cure rate of the fusion protein rLZ-8-HSA is significantly improved, which is unexpected to the inventor. According to the present invention, a preferred embodiment of the fusion protein rLZ-8-HSA for the treatment of thrombocytopenia is also provided. Accordingly, compared with a model group, an rLZ-8-HSA drug significantly stimulates proliferation of mouse platelets at a beginning of feeding, wherein a difference is extremely significant. In the mid-term of feeding, an effect thereof returns to a normal level. Sufficient effect is also observed in treatment of experimental animal models with thrombocytopenia caused by injection of anti-platelet serum.

Benefit effects of the present invention are as follows. According to the present invention, in vivo half-life of the fusion protein provided is significantly prolonged compared with the rLZ-8. Fusion protein constructed with gene fusion technologies will lower biological activity of a target protein after the fusion protein is produced. However, according to the present invention, the biological activity of the fusion protein rLZ-8-HSA has been experimentally proved to be significantly better than the biological activity of the rLZ-8 which is not fused. Besides, according to the present invention, fermentation of the fusion protein rLZ-8-HSA with the Pichia pastoris engineering strain is simple in technique, high in yield, single in expression product, and easy in purification, which provides favorable conditions for industrial production. There are problems such as an expression product is easy to be degraded during fermentation due to a Pichia pastoris expression system. Therefore, the present invention controls fermentation conditions, for greatly reducing degradation of the target products during Pichia pastoris fermentation expression, and increasing the yield. Through in vivo experiments, the present invention proves that compared with the rLZ-8 which is not fused, the in vivo half-life is significantly prolonged, while a lowest dosage and an onset time in the study of leukopenia treatment are also improved. Melanoma and liver tumor are researched as anti-tumor represents, and in vivo and in vitro experiments are respectively provided. According to the preferred embodiments of the melanoma and the liver tumor, therapeutic effects in the two experimental methods are significantly superior than the rLZ-8 not fused, which is unexpected to the inventor. According to the preferred embodiment of thrombocytopenia treatment, compared with the model group, the rLZ-8-HSA drug significantly stimulates proliferation of mouse platelets at the beginning of feeding, wherein the difference is extremely significant. In the mid-term of feeding, the effect thereof returns to the normal level, which is conventionally a best effect of the fusion protein of the rLZ-8.

**BRIEF DESCRIPTION OF THE DRAWINGS**

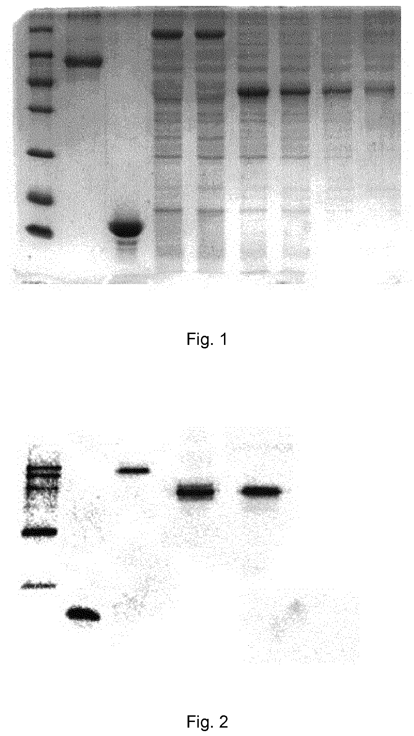

Fig. 1 illustrates inducing expression of different fusion proteins at 66h and 72h;
wherein a lane 1 sample is a protein marker; a lane 2 sample is a standard HSA; a lane 3 sample is an rLZ-8; a lane 4 sample is a supernatant of an rLZ-8-HSA after being induced for 72h; a lane 5 sample is a supernatant of the rLZ-8-HSA after being induced for 66h; a lane 6 sample is a supernatant of an rLZ-8-Fc1 after being induced for 72h; a lane 7 sample is a supernatant of the rLZ-8-Fc1 after being induced for 66h; a lane 8 sample is a supernatant of an rLZ-8-Fc4 after being induced for 72h; a lane 9 sample is a supernatant of the rLZ-8-Fc4 after being induced for 66h.

Fig. 2 is a Western Bolt identification map of different fusion proteins; wherein a lane 1 sample is a protein marker; a lane 2 sample is an rLZ-8; a lane 3 sample is an rLZ-8-HSA; a lane 4 sample is an rLZ-8-Fc1; a lane 5 sample is an rLZ-8-Fc4.

Fig. 3 is a chromatogram map of the fusion protein rLZ-8-HSA after being purified with a molecular sieve.

Fig 4 illustrates in vivo half-life comparison of different fusion proteins and the rLZ-8.

**DETAILED DESCRIPTION OF THE PREFERRED EMBODIMENT**

Preferred embodiment 1: rLZ-8 fusion protein engineering strains construction and expression

Construction: target fragments rLZ8-HSA, rLZ8-Fc1 and rLZ8-Fc4 sequences are respectively synthesized according to yeast codon preferences, and is stored in a puc57 plasmid; a primer comprising restriction enzyme cutting sites Stul and Kpnl is design; the primer is synthesized as follows:

(1) LZ-8-HSA: 5' CATAGGCCTTCTGATACTGCTTTGA 3'
5' CGGGGTACCGAATTCCTATTACA 3'

(2) LZ-8-Fc1: 5' GTTAGGCCTTCTGATACTGCTTTGA 3'
5' TAGGGTACCTCATTTACCAGGGG 3'

(3) LZ-8-Fc4: 5' CCGAGGCCTTCTGATACTGCTT 3'
5' GATGGTACCTCACGGAGCATGAG 3'

Obtaining the target fragments through PCR, wherein conditions for PCR are: firstly, 95°C for 30s; then 95°C for 30s, 58°C for 30s, and 72°C for 2min, wherein the above processes are repeated for 30 times; finally, 72°C for 10min, and standing by at 16°C.

Identifying by 1% agarose electrophoresis that the fragments are respectively at 2184bp (LZ-8-HSA), 1064bp (LZ-8-Fc1) and 774bp (LZ-8-Fc4); treating a pPICZα A carrier and the target fragments according illustrations of a GENEART Seamless Cloning and Assembly Kit, wherein a mole ratio of the carrier and the fragments is 1:3, wherein the carrier and the fragments are reacted with 5X buffer and 10X enzyme mixture for 30min; transforming 10ul connection product into colibacillus competence DH5 alpha; after being cultured at 37°C for 30min, coating the connecting product on an LB plate with bleomycin resistance; selecting a single bacterial colony is selected to be cultured at 37°C with a shaker for a night; centrifuging bacterial liquid with 12000g, and removing supernatant; extracting a recombinant plasmid with a plasmid small extraction kit, and testing a sequence thereof; using double enzyme digestion and electrophoresis for identifying whether a converter is correct; finally, using 5'AOX and 3'AOX primers for testing correctness of the sequence.

Linearizing the recombinant plasmid with Sacl enzyme at 37°C for about 1h; adding 33 times Pichia pastoris for inoculating to YPD at 30°C, and culturing with a 300rpm shaker for a night; enlarging to 500mL; when $OD_{600}$ reaches about 1.3, preparing yeast competence; ice-bathing for 30min and re-suspending with ice bath sterile water; centrifuging with 1500g at 4°C for 5min, and dividing into 80ul yeast competence per tube; adding 10ug linearizing plasmid; ice-bathing for 5min; transferring to a electro-transforming device and providing electro-transforming under 1.5kV, 50μF, 25mA; shaker-culturing at 30°C for 2h, a coating on an YPDS plate with bleomycin Zeocin resistance, and culturing at 30°C for 3 days.

screening: under an aseptic condition, selecting 20 mono-clones of each fusion protein on the YPDS plate with the bleomycin Zeocin resistance, and placing in 10mL YPD liquid culturing base for culturing at 30°C with a 300rpm shake flask for 12h; centrifuging with 1500g for 5min and removing supernatant; changing to a BMGY culturing base for culturing at 30°C with a 300rpm shake flask for 18h; centrifuging with 1500g for 5min and removing supernatant; changing to a BMGY (with 1% methanol) culturing base, and adding 1% methanol once per 24h; providing inducing expression for 72h; centrifuging with 1500g for 5min, and keeping supernatant at -20°C; quantitatively and qualitatively describing the expression of the target proteins through SDS electrophoresis and Western Bolt identification, so as to selecting high-expression engineering strains.

Preferred embodiment 2: purification techniques of fusion proteins

Because the fusion protein has a public LZ-8 structure, purification methods such as gravity column affinity chromatography, molecular sieve, immobilized metal chelate affinity chromatography (IMAC) method, hydrophobic interaction chromatography (HIC), anion exchange chromatography are studied according to characteristics of the structure, wherein specific methods are as follows.

The purification methods of the rLZ-8-Fc1 and the rLZ-8-Fc4 are as follows.

Microfiltration: centrifuging a fermentation liquid with 10000rpm for obtaining supernatant, then purifying (microfiltering) by a hollow fiber column with a hole diameter of 100Kd, so as to remove small molecular salts and sugars, for obtaining 8L yellow clear liquid comprising pigment, nucleic acid and protein.

rProtein A Gravi Trap gravity column affinity chromatography: preparing buffer solution A phase: 0.22M phosphate buffer solution, pH 7.7, and 0.15M sodium chloride; preparing buffer solution B phase: 0.1M citrate buffer solution, pH 4.0, 0.22μm vacuum filtration, ultrasonic degassing; balancing a chromatographic column with the A phase phosphate buffer solution, wherein a volume thereof is 10ml; respectively sampling 10ml pre-treated rLZ-8-Fc1 and rLZ-8-Fc4 fermentation liquid, and combining for two times; (sample treatment: 90ml the fermentation liquid and 10ml 10X the phosphate buffer solution, 0.22μm filtration and sterilized storage), washing the chromatographic column with the A phase phosphate buffer solution, eluting without combination; washing an eluting sample with the B phase citrate buffer solution, wherein a volume thereof is 10ml; waiting for 2min, and removing 1ml volume in the chromatographic column; receiving the samples with a 1.5ml centrifuging tube, and storing for testing; regenerating the chromatographic column after being used for 5 times, adding 5ml 6M guanidine hydrochloride, waiting for 2min, and washing the chromatographic column with the A phase.

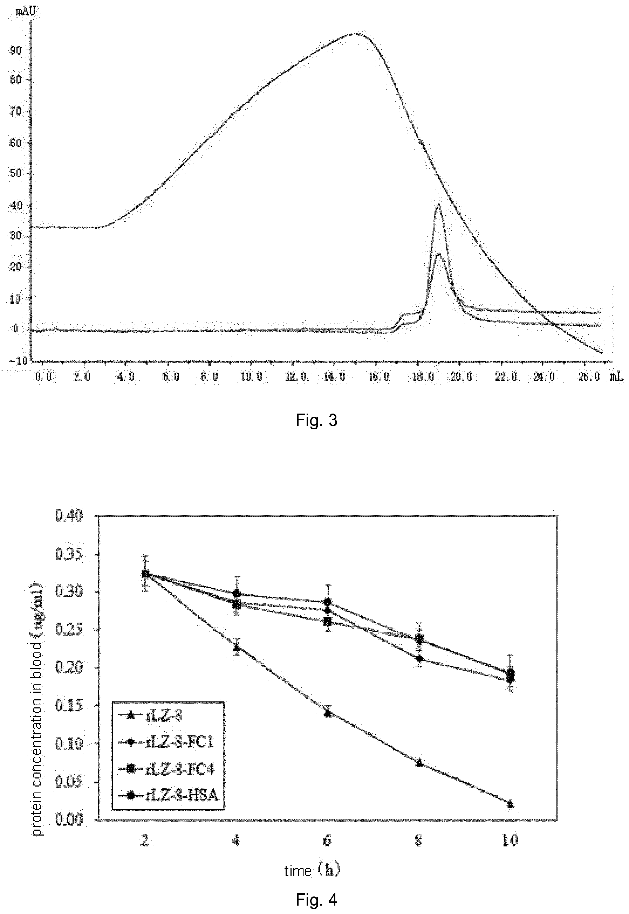

Molecular sieve chromatography: using a Superdex 75 filling column (GE, XK16/70, wherein an internal diameter thereof is 16mm, a height thereof is 70cm), wherein a filling height is 60cm; testing with 100μL 1% acetone, wherein a column efficiency is about 10000 according to a result; sampling protein with a flow rate of 2mg/mL and a concentration of 5mL, then eluting with pH 7.5, $NaH_2PO_4$-$Na_2HPO_4$ (50mM) buffer solution (illustrated in fig. 3); sampling at a collection peak, and providing electrophoresis and HPLC tests.

Results: after fine purification, a purity of the protein is above 99% according to the HPLC test, SDS-PAGE electrophoresis has a single strip.

A purification method of the rLZ-8-HSA comprises steps of:

step (1): purifying the rLZ-8-HSA with the Immobilized metal chelate affinity chromatography (IMAC) method, wherein a filler IMAC Sepharose 6Fast Flow 1 is purchased for GE; filling a XK50/30 column with a filling height of 15cm; replacing a storage liquid with pure water; passing 0.1M copper sulfate solution through a chromatography column, wherein a volume of the copper sulfate equals to a column volume, and washing copper ions which are not adsorbed with purified water, wherein a volume of the purified water is four times of the column volume; then balancing the chromatography column with a buffer solution A: 20mmol/L phosphate, 0.6mol/L sodium chloride, and pH 7.3, wherein a supernatant comprising the human serum albumin is added into the phosphate and the sodium chloride for forming 20mmol/L phosphate, 0.6mol/L sodium chloride, and pH 7.3; then sampling with an AKTA™ Purify chromatography system with a flow rate of 50ml/min; after sampling, washing with the buffer solution A until an absorption value reaches a basic point; eluting the target protein with a buffer solution B: 20mmol/L phosphate, 0.6mol/L sodium chloride, 0.3M iminazole and pH 7.5; collecting an eluting peak of the buffer solution B;

step (2): purifying with hydrophobic interaction chromatography (HIC), wherein the eluting peak of the buffer solution B collected in the step (1) is purified with hydrophobic interaction chromatography; filling a chromatography having a diameter of 5cm with a phenyl hydrophobic chromatography medium Phenyl Sepharose™ 6Fast Flow(high sub) (from GE), wherein a column height is 15cm; wherein before utilization, the medium is balanced with a buffer solution C: 50mmol/L phosphate, 0.5M sodium chloride, and pH 6.5, and a volume of the buffer solution C is 3 times of a column volume; adding deionized water into the buffer solution B comprising the target protein, which is obtained in the step (1), diluting the a concentration of 0.5M NaCl, and adjusting a pH value to 6.5 with phosphoric acid; sampling with the AKTA™ Purify chromatography system with a flow rate of 50ml/min; after sampling, eluting with the buffer solution C: 50mmol/L phosphate, 0.5M sodium chloride, and pH 6.5, wherein the volume of the buffer solution C is 2 times of the column volume; collecting a flow through peak and an eluting peak with the buffer solution C; eluting a combination portion with the hydrophobic chromatography column with the deionized water having a volume two times of the column volume, and discharging effluent liquid; adding collected liquid from the hydrophobic chromatography column into sodium tetraborate and calcium chloride solution with a final concentration of 0.1M, adjusting pH to 9.0 and treating for 0.5-24h, then centrifuging with 10000rpm for 20min, collecting supernatant, and desalinating with a MILLIPORE 10K ultrafiltration membrane; and

step (3): fining the sample with anion exchange chromatography, loading a Q Sepharose™ High Preformance filler into a chromatography with a diameter of 2.6cm and a height of 15cm, wherein a filling volume is 80ml; washing with the deionized water, wherein a volume thereof is two times of a column volume; then balancing with a buffer solution E (50mMpb, pH 7.0) having a volume five times of the column volume; after sampling, washing with the buffer solution E, wherein the volume thereof is tow times fo the column volume; then eluting with 0-0.5M NaCl with 10 times column volume gradient, and collecting a main peak.

Results: after fine purification, a purity of the protein is above 99% according to the HPLC test, SDS-PAGE electrophoresis has a single strip.

Preferred embodiment 3: comparison of different fusion proteins on promotion of mouse spleen cell proliferation

Testing effects of the fusion proteins on mouse spleen cell proliferation by a WST-1 method, which illustrates strength of biological activity, wherein according to the present invention, BALB/c female mice are used, whose weight is controlled at 20-22g; executing the mice by stretching necks thereof, taking out spleens under a sterile condition, and putting into a plate with 5ml DMEM comprising 10% calf serum; cracking the spleens with tweezers, filtering tissue suspension with gauze for removing tissue blocks, and preparing cell suspension of spleen cells; adding $100\mu l$ the tissue suspension into $900\mu l$ 2% glacial acetic acid for counting with a microscope; adjusting a cell concentration to $5\times10^6$/ml with DMEM comprising 2% calf serum; respectively preparing the fusion proteins and the rLZ-8 with same molar concentration gradient, wherein there are 3 gradient concentrations, each concentration occupies 9 wells with $100\mu l$ per well; adding tissue suspension with a concentration of $5\times10^6$/ml with $100\mu l$ per well; shaking for evenly mixing, and then putting into a 37°C, 5% $CO_2$ incubation device to incubate for 24h; after incubation, adding WST-1 with $20\mu l$ per well; putting into the 37°C, 5% $CO_2$ incubation device to incubate for 3h, then testing $OD_{450}$ (with a BIO-RAD); wherein results are listed in table 1.

Table 1: Biological activity of different fusion proteins on promotion of cell proliferation (x±s n=9)

| Protein Concentration | rLZ-8 | rLZ-8- Fc1 | rLZ-8-Fc4 | rLZ-8-HSA |
|---|---|---|---|---|
| $5.0\times10^{-9}$mol/L | 0.739±0.23 | 0.543±0.26 | 0.452±0.43 | 0.936±0.31[*] |
| $10.0\times10^{-9}$mol/L | 1.197±0.31 | 0.937±0.29 | 0.840±0.27 | 1.680±0.38[*] |
| $15.0\times10^{-9}$mol/L | 1.567±0.27 | 1.114±0.31 | 0.929±0.21 | 2.373±0.35[*] |

Note: comparison with rLZ-8, $*p < 0.05$

Referring to table 1, with increase of the protein concentration, an effect of the fusion proteins on spleen cell proliferation also increases. With the same protein concentration, it can be concluded from proliferation effect comparison of the different fusion protein and the rLZ-8 that the rLZ-8-HSA is better than the rLZ-8 in promotion of the spleen cell proliferation, and there is a significant difference, which is statistically significant. However, promotion effects of the rLZ-8-Fc1 and the rLZ-8-Fc4 on the mouse spleen cell proliferation is significantly decreased. According to experimental results, active spots are not affected by fusion of LZ-8 and the HSA, while after fusion of the LZ-8 and IgG-Fc1 or IgG-Fc4, the protein activity is decreased, which hinders LZ-8 activity.

Preferred embodiment 4: half-life tests of different fusion proteins

Utilizing BALB/c mice weighing about 18-22g in experiments, wherein each group comprises 10 mice; intravenously injecting 100g/kg (judging from LZ-8 dosage) the fusion protein, respectively sampling blood after 2, 4, 6, 8 and 10 hours after injection, drawing a curve of medicine concentration per time (illustrated in fig. 4) with the results obtained, wherein it is indicated by the experiment results that the half-life of the fusion protein is significantly extended (P < 0.0001) according to the rLZ-8 which is not fused, a concentration thereof in blood is greatly prolonged, and the half-life of the rLZ-8 in the mouse is improved.

Preferred embodiment 5: research of fusion protein rLZ-8-HSA on treatment of

Utilizing Wistar rats in the experiments, wherein 18 rats weighing about 100g are utilized. A method for preparing reagents comprises steps of: dissolving the rLZ-8 in sterile saline, and diluting into $60\mu g/kg$, $30\mu g/kg$ and $15\mu g/kg$

dosage groups; dissolving the fusion protein rLZ-8-HSA in the sterile saline, and diluting into 60μg/kg, 30μg/kg and 15μg/kg (judging from the LZ-8 dosage) dosage groups; diluting GenLei®Scimax® [recombinant human granulocyte colony-stimulating factor injection (rhG-CSF)], batch number: 20130403, 75μg/vial, into 13.5μg/ml and 0.1ml per rat with the sterile saline; diluting cyclophosphamide (CP) injection, batch number 13020225, 200mg/vial, into 20mg/ml and 0.1ml per rat with the sterile saline, or 20mg/kg.

The experiment has an rLZ-8 low-dosage group, an rLZ-8 middle-dosage group, an rLZ-8 high-dosage group, an rLZ-8-HSA low-dosage group, an rLZ-8-HSA middle-dosage group, an rLZ-8-HSA high-dosage group, and a positive medicine control group (utilizing the GenLei®Scimax®). The rats of each the group are injected with the cyclophosphamide in tail vein for three days except that the sterile saline is given to the normal control group, the dosage is 20mg/ml and 0.1ml per rat. On the third day, the blood is sampled from the tail vein, and the leukocytes are counted by a cytoanalyzer. After successful modeling, the rats of each the group are respectively treated with the rLZ-8, the three kinds of the fusion proteins, or the positive medicine (the GenLei®Scimax®) with the corresponding dosage, and the equal sterile saline is given to the rats of the normal control and a CP group. The blood is sampled from the tail vein on the first, third and seventh treatment days, and the leukocytes are counted by the cytoanalyzer. Medicine efficacy is analyzed according to a number difference of the leukocyte between before and after the treatment.

Table 2: effects of rLZ-8 on rats models with leucopenia (n = 10)

| group | leukocyte number | on the first treatment day | on the third treatment day | on the seventh treatment day |
|---|---|---|---|---|
| normal control | $14.11\times10^9.L^{-1}$ | $14.36\times10^9.L^{-1}$ | $13.8\times10^9.L^{-1}$ | $12.13\times10^9.L^{-1}$ |
| CP control | $5.1\times10^9.L^{-1}$ | $5.3\times10^9.L^{-1}$ | $5.8\times10^9.L^{-1}$ | $9.27\times10^9.L^{-1}$ |
| GenLei® Scimax® | $4.55\times10^9.L^{-1}$ | $6.4\times10^9.L^{-1}$ | $11.83\times10^9.L^{-1*}$ | $11.17\times10^9.L^{-1}$ |
| rLZ-8 (low-dosage) | $3.71\times10^9.L^{-1}$ | $4.6\times10^9.L^{-1}$ | $9.3\times10^9.L^{-1*}$ | $11.2\times10^9.L^{-1}$ |
| rLZ-8 (middle-dosage) | $3.12\times10^9.L^{-1}$ | $5.1\times10^9.L^{-1}$ | $9.7\times10^9.L^{-1*}$ | $12.78\times10^9.L^{-1}$ |
| rLZ-8 (high-dosage) | $4.09\times10^9.L^{-1}$ | $5.4\times10^9.L^{-1}$ | $9.6\times10^9.L^{-1*}$ | $14.5\times10^9.L^{-1}$ |
| rLZ-8-HSA (low-dosage) | $3.11\times10^9.L^{-1}$ | $8.5\times10^9.L^{-1*}$ | $10.8\times10^9.L^{-1**}$ | $13.2\times10^9.L^{-1}$ |
| rLZ-8-HSA (middle-dosage) | $2.89\times10^9.L^{-1}$ | $9.2\times10^9.L^{-1*}$ | $11.98\times10^9.L^{-1**}$ | $13.4\times10^9.L^{-1}$ |
| rLZ-8-HSA (high-dosage) | $3.33\times10^9.L^{-1}$ | $8.4\times10^9.L^{-1*}$ | $14.4\times10^9.L^{-1**}$ | $15.5\times10^9.L^{-1}$ |

Note: comparison with CP control group, $*p < 0.05$, $**p < 0.01$

It is illustrated in table 2 that on the first treatment day, the leukocyte number of the rats of the rLZ-8-HSA groups is significantly increased in comparison with the rats of the CP group, and on the seventh treatment day, the leukocyte number basically approaches to a normal level. On the first treatment day, the leukocyte number of the rats of the rLZ-8-HSA groups is significantly increased in comparison with the rats of the GenLei®Scimax® control group, and on the seventh treatment day, the leukocyte number basically approaches to the normal level. It is emphasized that when compared with the rLZ-8 groups with the same dosage, the rLZ-8-HSA groups have a sufficient leukocyte proliferation effect from the first treatment day, wherein the leukocyte number is about 2 times more than the leukocyte number of the rLZ-8 groups; with a same treatment period, the leukocyte proliferation effect of the rLZ-8-HSA low-dosage group is superior to the leukocyte proliferation effect of the rLZ-8 high-dosage group and other rLZ-8 groups.

Preferred embodiment 6: inhibition effect of fusion protein rLZ-8-HSA on melanoma

In vitro experiment: testing the inhibition effect of the fusion protein rLZ-8-HSA on the melanoma through a WST-1 method, preparing tissue suspension of the melanoma; adding 100μl the tissue suspension into 900μl 2% glacial acetic acid and counting with a microscope; adjusting a tissue concentration to $2\times10^5$/ml with DMEM comprising 2% calf serum; respectively preparing the fusion protein rLZ-8-HSA and the rLZ-8 with same molar concentration gradient, wherein there are 3 gradient concentrations, each concentration occupies 9 wells with 100μl per well; adding tissue suspension with a concentration of $2\times10^5$/ml with 100μl per well; shaking for evenly mixing, and then putting into a 37°C, 5% $CO_2$ incubation device to incubate for 24h; after incubation, adding WST-1 with 20μl per well; putting into the 37°C, 5% $CO_2$ incubation device to incubate for 3h, then testing $OD_{450}$ (with a BIO-RAD); wherein results are listed in table 3.

Table 3: inhibition effect of fusion protein rLZ-8-HSA on melanoma (x±s, n=9)

| Protein Concentration | rLZ-8 | rLZ-8-HSA |
|---|---|---|
| $5.0 \times 10^{-9}$ mol/L | 0.427±0.44 | 0.411±0.31[*] |
| $10.0 \times 10^{-9}$ mol/L | 0.372±0.21 | 0.212±0.29[*] |
| $15.0 \times 10^{-9}$ mol/L | 0.302±0.24 | 0.118±0.11[**] |

Note: comparison with rLZ-8, *$p < 0.05$, **$p < 0.05$

In vivo experiment: firstly, establishing a mouse tumor mould, wherein mouse melanoma cells B16-F10 is cultured with the DMEM comprising 10% fetal calf serum at 37°C in a $CO_2$ incubation device; slowly subcutaneously injecting 200μl B16-F10 cell suspension (comprising $1 \times 10^7$ cells) with a 1ml injector at mouse dorsal-ventral skin, so as to establish a mouse transplanted tumor mould.

Group arrangement and treatment method: 24h after injecting the tumor cells, injecting at tail veins for rLZ-8 groups (123μg/kg, 246μg/kg and 492μg/kg), rLZ-8-HSA groups (123μg/kg, 246μg/kg and 492μg/kg judging from the LZ-8), and a dacarbazine group (2.5mg/kg) or a normal saline group, wherein the rLZ-8 and the rLZ-8-HSA are injected once a day, the dacarbazine is continually injected for 5 days, and then injected again after 3 weeks, wherein a treatment cycle comprises 28 days; during experiment, observing living states of the mouse, weighing once every seven days, and sampling tail vein blood of the mouse once every 2 weeks; separating tumor bodies at an ending of the experiment, weighing the tumor bodies and recording; calculating an inhibition rate of the treating medicines on in situ tumor growth according to a formula that a tumor inhibition rate = (an average tumor weight of the normal saline group - an average tumor weight of the treated group) / an average tumor weight of the normal saline group.

Referring to experimental results: after weighing the tumor bodies, the average tumor weight of each group is calculated; according to table 4, it is illustrated that after 28 days, a tumor weight of a rLZ-8-HSA high-dosage group is less than tumor weights of other groups; within rLZ-8 groups or rLZ-8-HSA groups, the more a concentration of the medicine is, the less the tumor weight will be. Difference between the rLZ-8-HSA groups and a negative control group is extremely significant. Difference between the rLZ-8 low-dosage group, middle-dosage group and the high-dosage group is also significant (n=10, P<0.05). Difference between the rLZ-8-HSA low-dosage group, middle-dosage group and the high-dosage group is also significant (n=10, P<0.05).

Table 4: effect of rLZ-8 on B16-F10 in situ tumor weight

| group / feeding method | animal quantity | continually feeding for 28 days | |
|---|---|---|---|
| | | tumor weight (g) | tumor inhibition rate(%) |
| normal saline | 10 | 1.24±0.53 | —— |
| dacarbazine | 10 | 0.52±0.12** | 62.57±0.27 |
| rLZ-8 low-dosage | 10 | 0.53±0.21**## | 56.72±0.41 |
| rLZ-8 middle-dosage | 10 | 0.43±0.17**## | 64.91±0.77 |
| rLZ-8 high-dosage | 10 | 0.25±0.04** | 83.04±0.51## |
| rLZ-8-HSA low-dosage | 10 | 0.35±0.11**## | 71.77±0.31## |
| rLZ-8-HSA middle-dosage | 10 | 0.27±0.14**## | 78.22±0.47## |
| rLZ-8-HSA high-dosage | 10 | 0.13±0.05** | 89.51±0.88## |

Note: comparison with control group, *$P < 0.05$, **$P < 0.01$, wherein the experiment is repeated for three times, total results are trends to be the same, and the table 4 is only the result of one experiment; comparison with the dacarbazine group, #$P < 0.05$, ##$P < 0.01$, wherein the experiment is repeated for three times, total results are trends to be the same, and the table 4 is only the result of one experiment.

Preferred embedment 7: inhibition effect of fusion protein rLZ-8-HSA on mouse S180 Ehrlich ascites tumor

In vitro experiment: testing the inhibition effect of the fusion protein rLZ-8-HSA on the mouse S180 Ehrlich ascites tumor through the WST-1 method, preparing tissue suspension of the mouse S180 Ehrlich ascites tumor; adding 100μl the tissue suspension into 900μl 2% glacial acetic acid and counting with a microscope; adjusting a tissue concentration to $2 \times 10^5$/ml with DMEM comprising 2% calf serum; respectively preparing the fusion protein rLZ-8-HSA and the rLZ-8 with same molar concentration gradient, wherein there are 3 gradient concentrations, each concentration occupies 9 wells with 100μl per well; adding tissue suspension with a concentration of $2 \times 10^5$/ml with 100μl per well; shaking for evenly mixing, and then putting into a 37°C, 5% $CO_2$ incubation device to incubate for 24h; after incubation, adding WST-1 with 20μl per well; putting into the 37°C, 5% $CO_2$ incubation device to incubate for 3h, then testing $OD_{450}$ (with a BIO-RAD); wherein results are listed in table 5.

Table 5: inhibition effect of fusion protein rLZ-8-HSA on mouse S180 Ehrlich ascites tumor (x±s, n=9)

| Protein Concentration | rLZ-8 | rLZ-8-HSA |
|---|---|---|
| $5.0 \times 10^{-9}$ mol/L | 0.453±0.23 | 0.408±0.21[*] |
| $10.0 \times 10^{-9}$ mol/L | 0.366±0.31 | 0.233±0.18[*] |
| $15.0 \times 10^{-9}$ mol/L | 0.345±0.27 | 0.127±0.15[**] |

Note: comparison with the rLZ-8 groups, *$p < 0.05$, **$p < 0.05$

In vivo experiment: experimental material: mice weighing 18-22g from Laboratory Animal Center of Jilin University; Ehrlich ascites tumor cell strains are provided by a lab of the inventor; cytoxan (CTX) is from Jiangsu Hengrui Medicine Co., Ltd, whose batch number is 06101921; S180 ascites tumor and soild tumor experimental groups respectively comprises a normal control group, a negative control group, a positive control group, an rLZ-8 low-dosage group ($0.25$mg·kg$^{-1}$), an rLZ-8 middle-dosage group ($0.5$mg·kg$^{-1}$), an rLZ-8 high-dosage group ($1$mg·kg$^{-1}$), an rLZ-8-HSA low-dosage group ($0.25$mg·kg$^{-1}$), an rLZ-8-HSA middle-dosage group ($0.5$mg·kg$^{-1}$), and an rLZ-8-HSA high-dosage group ($1$mg·kg$^{-1}$), wherein dosages of the rLZ-8-HSA groups are judged from LZ-8 dosage, wherein each group comprises 10 mice.

Experimental method: experimental method of subcutaneous inhibition of the S180 tumor: selecting well-grown S180 cells, diluting by sterile saline with a proper amount for preparing tumor cell suspension, whose cells are counted as $10^7$L$^{-1}$, subcutaneous injecting at a right armpit of each mouse with a dosage of 20ml (except for the normal control group); after 24h, treating the mice; abdominally injecting normal saline to the normal and negative control groups with a dosage of 0.2ml per mouse per day; abdominally injecting 20 mg·kg$^{-1}$ cyclophosphamide to the positive control group with a dosage of 0.2ml per mouse per day; providing tail vein injection to the rLZ-8 groups with corresponding dosages, 0.2ml per mouse per day and lasting for 10 days; before injection and 10d after injection, sampling blood from mouse orbital venous plexus, and counting white blood cells by a clinical laboratory of First Hospital of Jilin university; on a next day after injection, executing all the mice by cervical dislocation, disserting and taking out tumor blocks, weighing and calculating a tumor inhibition rate according a following formula:

$$\text{tumor inhibition rate (\%)} = (\text{average tumor weight of control groups} - \text{average tumor weight of experimental groups}) / \text{average tumor weight of control groups} \times 100\%.$$

Experimental result: experimental results of subcutaneous inhibition of the S180 tumor: referring to table 6, the three rLZ-8 groups are all able to inhibit S180 growth, wherein tumor inhibition rates thereof are respectively 16.8%, 25.7% and 45.5%. Compared with the negative control group, tumor weights of the rLZ-8 groups have significant difference ($P < 0.01$). Compared with the negative control group, tumor weights of the rLZ-8-HSA groups have significant difference ($P < 0.01$). Meanwhile, compared with the rLZ-8 groups, tumor weights of the rLZ-8-HSA groups also have significant difference ($P < 0.01$).

Table 6: inhibition effect of rLZ-8 on mouse transplanted tumor S180 (x±s, n=10)

| Group | Tumor weight (g) | Inhibition rate (%) |
|---|---|---|
| negative control | 1.01±0.03 | - |
| CTX | 0.36±0.02* | 64.3 |
| rLZ-8 low-dosage | 0.83±0.03* | 17.8 |
| middle-dosage | 0.74±0.02* | 26.7 |
| rLZ-8 high-dosage | 0.57±0.03* | 43.5 |
| rLZ-8-HSA low-dosage | 0.73±0.03*# | 27.7 |
| rLZ-8-HSA middle-dosage | 0.44±0.05*# | 56.4 |

(continued)

| Group | Tumor weight (g) | Inhibition rate (%) |
|---|---|---|
| rLZ-8-HSA high-dosage | 0.21±0.08**## | 29.3 |

Note: comparison with the negative control group, *P < 0.05, **P < 0.01; comparison with the rLZ-8 groups, #P < 0.05, ##P < 0.01.

Preferred embodiment 8: pharmacodynamics experiment of fusion protein rLZ-8-HSA on treatment of mouse thrombocytopenia caused by cyclophosphamide

Experimental medicine: preparing a 19.25μg·kg$^{-1}$ and a 9.625μg·kg$^{-1}$ dosage groups of the recombinant *ganoderma* immunoregulatory protein (rLZ-8) with sterile injection water, and 0.2ml per mouse; and preparing a 19.25μg·kg$^{-1}$ and a 9.625μg·kg$^{-1}$ dosage groups (judging from the LZ-8) of the fusion protein rLZ-8-HSA of the recombinant *ganoderma* immunoregulatory protein.

Positive control medicine: thrombopoietin (THPO) from Shenyang 3SBio Inc., with a dosage of 770μg·kg$^{-1}$/d and 0.2ml per mouse.

Chemotherapy medicine: cyclophosphamide (Cy) from Jiangsu Hengrui Medicine Co., Ltd, whose batch number is 12112121, 200mg/bottle; preparing into 100mg·kg$^{-1}$, 0.2ml per mouse with sterile injection water.

Platelet dilution: urea: 1.3g; sodium citrate: 0.5g; formaldehyde: 0.1ml; adding distilled water to 100ml for mixing, then filtering for further utilization.

Experimental method: dividing experimental animals into 5 groups, wherein each group comprises 10 mice with 5 males and 5 females; except for a normal control group (injected with normal saline having an equal volume), abdominally injecting the cyclophosphamide to the mice with a dosage of 100mg·kg$^{-1}$, 0.2ml per mouse per day, and lasting for three days; when a platelet quantity decreased to below $300×10^9$/L, subcutaneously injecting the rLZ-8, the fusion protein rLZ-8-HAS (19.25μg·kg$^{-1}$, 9.625μg·kg$^{-1}$, 0.2ml·per mouse per day) and the positive medicine (THPO 770μg·kg$^{-1}$, 0.2ml·per mouse per day) according to the above groups with corresponding dosages, and injecting the normal saline having the equal volume to the normal control group and a CP group; respectively sampling mouse tail vein blood 3d, 7d and 14d after treatment, counting platelets with high power lens, wherein results thereof are listed in table 7.

Experimental result: compared with the CP group, platelets of the mice of the treated groups significantly increased on the third day, and returned to normal at the seventh day, wherein significant difference exists (p < 0.05); platelets of the mice of the rLZ-8-HSA groups significantly increased, and returned to normal at the third day, wherein significant difference exists (p < 0.05) compared with the CP group, which is statistically significant.

Table 7: experimental results of rLZ-8 on treatment of mouse thrombocytopenia (x±s, n=10) (unit: *10$^9$/L)

| Group | before | after | 3$^{rd}$ day | 7$^{th}$ day | 14$^{th}$ day |
|---|---|---|---|---|---|
| normal control | 838±0.11 | 946±0.18 | 870±0.23 | 760±0.31 | 965±0.70 |
| CP | 850±0.34 | 239±0.12 | 256±0.93 | 301±0.30 | 323±0.67 |
| THPO | 851±0.34 | 284±0.19 | 390±0.38 | 420±0.32 | 689±0.65 |
| rLZ-8 (9.625μg·kg$^{-1}$) | 839±0.40 | 201±0.41 | 483±0.45 | 585±0.78 | 898±0.61* |
| rLZ-8 (19.25μg·kg$^{-1}$) | 946±0.45 | 226±0.78 | 501±0.48 | 680±0.28 | 1021±0.31** |
| rLZ-8-HSA (9.625μg·kg$^{-1}$) | 843±0.65 | 261±0.52 | 624±0.43* | 697±0.43* | 929±0.61** |
| rLZ-8-HSA (19.25μg·kg$^{-1}$) | 852±0.82 | 246±0.36 | 760±0.38* | 978±0.67* | 1035±0.78** |

Note: there is significant different when compared with the CP group, wherein *p < 0.05, **p < 0.01

Preferred embodiment 9: rLZ-8-HSA anti-tumor composition preparation

1) According to the above pharmacological experiments, it is proved that an anti-tumor effect of the rLZ-8-HSA is extremely effective on maintaining body white blood cell level without toxic side effect. Therefore, it is considered that the rLZ-8-HSA is suitable for medicine and is safe.

2) According to the present invention, application of the rLZ-8-HSA as an anti-tumor medicine is able to be an oral form and a parenteral form. A dosage thereof depends on factors such as symptoms, age, and weight. For adults with the oral form, 10-1000mg a time and several times a day; for the parenteral form, 10-100mg a time and several times a day.

3) The present invention comprises oral tablets and pill capsules (comprising hard and soft capsules), wherein the above forms comprises the rlz-8 and at least one inert diluent (e.g. lactose, mannitol, glucose, starch, polyvinyl pyrrolidone). Besides the inert diluent, pharmaceutically acceptable additives such as lubricants, disintegrating agent, and stabilizer are also able to be added. If necessary, tablets or pills may be coated with a gastric or enteric soluble material by one or more layers. Non-parenteral injection comprises the rLZ-8-HSA and at least one inert diluent (such as distilled water, saline solution). Or, the rLZ-8-HSA may be prepared into freeze-dried powders, which is dissolved in the inert diluent for injection before utilization.

(1) Preparation 1

Dissolving 1000mg the rLZ-8-HSA in 100ml sterile saline, evenly mixing, and dividing the rLZ-8-HSA into drug bottles with a concentration of 10mg/ml/tube, sealing and sterilizing for forming a final product; wherein other events are in line with injection liquid requirements of Pharmacopoeia of the People's Republic of China, 2010.

(2) Preparation 2

Preparing capsules with 100g the rLZ-8-HSA and 0.5kg pharmaceutical starch with common capsule preparation technologies and devices, wherein the rLZ-8-HSA is divided into 10mg/grain, wherein other events are in line with capsule requirements of Pharmacopoeia of the People's Republic of China, 2010.

(3) Preparation 3

Preparing pills with 100g the rLZ-8-HSA, 560g microcrystalline cellulose, 380g lactis anhydrous and 200g magnesium stearate with common pill preparation technologies and devices, wherein the rLZ-8-HSA is divided into 10mg/pill, wherein other events are in line with pill requirements of Pharmacopoeia of the People's Republic of China, 2010.

(4) Preparation 4

Preparing oral liquid with a proper amount of the rLZ-8-HSA with common oral liquid preparation technologies and devices, which is in line with oral liquid requirements of Pharmacopoeia of the People's Republic of China, 2010.

## Claims

1. A fusion protein (rLZ-8-HSA) of a recombinant *ganoderma* immunoregulatory protein and a human serum albumin, wherein a C-terminal of a *ganoderma* immunoregulatory protein is connected to the human serum albumin through a connecting peptide, and an amino acid sequence of the fusion protein is SEQ ID NO. 1.

2. The fusion protein according to claim 1 for use in treating leukopenia caused by chemotherapy.

3. The fusion protein according to claim 1 for use in treating melanoma and liver tumor.

4. The fusion protein according to claim 1 for use in treating thrombocytopenia.

5. A pharmaceutical composition, comprising a fusion protein as recited in claim 1 and any pharmaceutically acceptable additive.

6. The composition according to claim 5, wherein the composition is in an oral form and a parenteral form; wherein the oral form comprises oral liquid, tablets, pills and capsules; and wherein the parenteral form comprises topical medicines and injections.

## Patentansprüche

1. Fusionsprotein (rLZ-8-HSA) eines rekombinanten immunregulatorischen *Ganoderma*-Proteins und eines Humanserumalbumins, wobei ein C-Terminus eines immunregulatorischen *Ganoderma*-Proteins mit dem Humanserumalbumin durch ein verbindendes Peptid verbunden ist und eine Aminosäuresequenz des Fusionsproteins SEQ ID NO. 1 hat.

2. Fusionsprotein nach Anspruch 1 zur Verwendung bei der Behandlung von durch Chemotherapie verursachter Leukopenie.

3. Fusionsprotein nach Anspruch 1 zur Verwendung bei der Behandlung von Melanom und Lebertumor.

4. Fusionsprotein nach Anspruch 1 zur Verwendung bei der Behandlung von Thrombozytopenie.

5. Pharmazeutische Zusammensetzung, umfassend ein Fusionsprotein nach Anspruch 1 und beliebige pharmazeutisch akzeptable Zusatzstoffe.

6. Zusammensetzung nach Anspruch 5, wobei die Zusammensetzung in einer oralen Form und einer parenteralen Form vorliegt; wobei die orale Form orale Flüssigkeit, Tabletten, Pillen und Kapseln umfasst; und wobei die parenterale Form topische Medikamente und Injektionen umfasst.

**Revendications**

1. Protéine de fusion (rLZ-8-HSA) associant une protéine immunorégulatrice de *ganoderma* recombinante à une sérum albumine humaine, dans laquelle une extrémité C-terminale d'une protéine immunorégulatrice de *ganoderma* est liée à la sérum albumine humaine par un peptide de liaison, et une séquence d'acides aminés de la protéine de fusion est SEQ ID NO : 1.

2. Protéine de fusion selon la revendication 1 pour son utilisation dans le traitement de la leucopénie provoquée par une chimiothérapie.

3. Protéine de fusion selon la revendication 1 pour son utilisation dans le traitement du mélanome et d'une tumeur du foie.

4. Protéine de fusion selon la revendication 1 pour son utilisation dans le traitement de la thrombopénie.

5. Composition pharmaceutique, comprenant une protéine de fusion selon la revendication 1 et tout additif pharmaceutiquement acceptable.

6. Composition selon la revendication 5, dans laquelle la composition se trouve sous une forme orale et une forme parentérale ; dans laquelle la forme orale comprend un liquide oral, des comprimés, des pilules et des capsules ; et dans laquelle la forme parentérale comprend des médicaments topiques et des injections.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201110222012
- CN ZL200810050206X

- WO 201310479016 A

**Non-patent literature cited in the description**

- *Pharmacopoeia of the People's Republic of China,* 2010